**Europäisches Patentamt**

**European Patent Office**

**Office européen des brevets**

(19)

(11) Publication number: **0 149 982**
**A2**

(12) # EUROPEAN PATENT APPLICATION

(21) Application number: **84830332.7**

(22) Date of filing: **06.12.84**

(51) Int. Cl.⁴: **A 61 M 1/10**

(30) Priority: **14.12.83 IT 3634183 U**

(43) Date of publication of application:
**31.07.85 Bulletin 85/31**

(84) Designated Contracting States:
**AT BE CH DE FR GB IT LI NL SE**

(71) Applicant: **De Virgiliis, Giuseppe**
**Via Montepulciano 62**
**I-00182 Roma(IT)**

(71) Applicant: **Vanin, Mario**
**Via Gallonio 9**
**I-00161 Roma(IT)**

(72) Inventor: **De Virgiliis, Giuseppe**
**Via Montepulciano 62**
**I-00182 Roma(IT)**

(72) Inventor: **Vanin, Mario**
**Via Gallonio 9**
**I-00161 Roma(IT)**

(74) Representative: **Tonon, Gilberto et al,**
**c/o Società Italiana Brevetti Piazza Poli 42**
**I-00187 Roma(IT)**

(54) A pumping device for extra-corporeal circulation of biological fluids, in particular blood.

(57) A pumping device for the extra-corporeal circulation of biological fluids, in particular blood, comprising a positive displacement pump system connected with a circuit of a sterile and biologically compatible material, which comprises a flexible suction tube, a flexible delivery tube in communication with a therapeutic treatment apparatus, a manifold to which the ends of said suction and delivery tubes are connected, connecting means to the body of a patient for the inlet into said suction tube and for the outlet from said delivery tube of said biological fluid, and means for the unidirectional flow of said biological fluid in said suction and delivery tubes.

FIG.2

EP 0 149 982 A2

De Virgiliis, Giuseppe

Vanin, Mario

## "A pumping device for extra-corporeal circulation of biological fluids, in particular blood."

Presently, according to the state of the art related to the extra-corporeal circulation of biological fluids for their treatment or for particular surgical operations, there are commonly used peristaltic pumps, generally of the roller type, which, by compression deformation of a flexible conduit made with biologically compatible material, allow the forced transit of these fluids so that they are made to pass through a suitable therapeutical apparatus for performing said treatment or in such a way that the natural circulation of said fluids is substituted by a similar artificial one in the event of said surgical operations.

The biological fluid that normally is subjected to a therapeutical treatment with the above mentioned art, for instance through oxygenators, artificial kidneys, cartridges of activated carbon, or directed out of its natural circulation bed for performing surgical operations, for example heart surgery, is blood that shows chemical-physical characteristics such that in performing extra-corporeal circulation, one is compelled to fulfil at least three conditions:

1. sterility of the circuit where the flow occurs;
2. biological compatibility of the materials that constitute the circuit;

3.  absence of damaging mechanisms that traumatize the cellular components of the blood (red corpuscles, leucocytes, platelets).

The above said peristaltic pumps, up to now commonly adopted as said before, for the extra-corporeal circulation of blood, surely satisfy at least two of the three above mentioned conditions and precisely the first and the second ones concerning the sterility and the biological compatibility of the circuit. Insofar as the third requirement is concerned, instead, i.e. the absence of a traumatizing action, said pumps show a serious inconvenience deriving from the fact that their mechanical action, besides being exerted on the liquid part of the blood, undesirably operates also upon the corpuscular part of the same, and in particular on the red corpuscles and on the platelets through a crushing-mechanism.

It is true, indeed, that the above said damages caused on the blood by the pumps in question are of a modest entity when they may be referred to a sporadic and occasional treatment, but in the case of a prolonged therapy such as the case of haemodialysis, the number of hours-year (more than 600) of treatment creates a situation of not neglectable damage that by no means can be disregarded.

The object of the present invention is to provide a pumping device for the extra-corporeal circulation of biological fluids, with particular reference to blood, that satisfying, of course, the sterility and biological compatibility requirements of the circuit, eliminates almost entirely the above referred traumatizing action of the known devices of this kind in respect of the involved biological fluid.

The present invention will be better illustrated in the following disclosure of preferred embodiments of the same, given by way of example and not as a limitation, with reference to the single attached drawing, wherein:

figure 1 is a schematic   view, with omitted parts, of a pumping device according to the invention; and

figure 2 is a schematic view, with omitted parts, of a modification of the pumping device of figure 1.

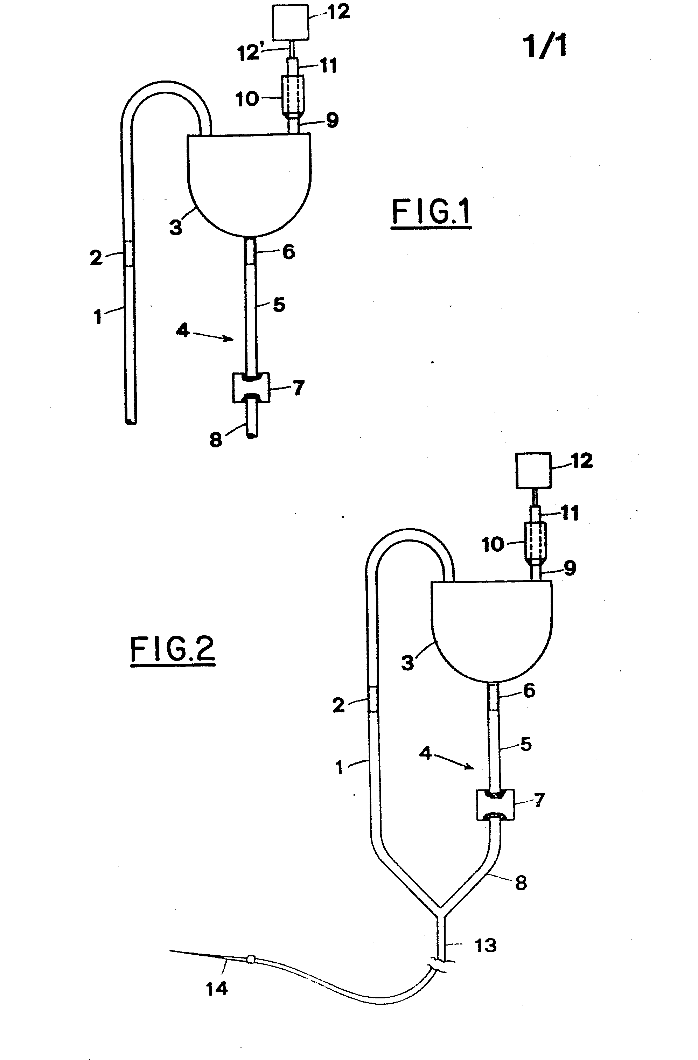

With reference to figure 1, the pumping device for the extra-corporeal circulation of biological fluids according to the present invention to be used, for instance, for a haemodialysis treatment, comprises a flexible suction tube 1,   provided with a first check valve 2, one end of said tube   being connected with the top of a manifold 3 while the other end is fastened to a first needle-tube (not shown), a flexible delivery tube, generally shown in 4, comprising a first portion 5 which has one end connected to the bottom of the manifold 3 through a second check valve 6 and the other end connected to the inlet of a treatment apparatus 7, in this case a suitable filter for scrubbing of blood, and a second portion 8 one end of which is connected with the outlet of the treatment apparatus 7 while the other end terminates with a second needle-tube (not shown).

The manifold 3 is connected at its top, in fluid communication, through a flexible tube 9 to a positive-displacement pump system, comprising a syringe-type instrument consisting of a cylinder 10 provided with a piston 11.

The above said components, all manufactured with sterile and biologically compatible materials, form in their whole a disposable assembly, for the extra-corporeal circulation of blood in haemodialysis with the related scrubbing treatment.

For performing the above said circulation, said positive-displacement pump system comprises a driving device schematically shown in 12, i.e. a suitable motor which in a conventional manner imparts a reciprocating motion to a rod 12'.releasably connected with the top of the piston 11 of the syringe-type instrument so that the piston 11 is drawn in reciprocating motion.

In operation, for said haemodialysis treatment the first needle-tube at the end of the suction tube 1 and the second needle-tube at the free end of the second portion 8 of the delivery tube 4 are inserted in the arm of the patient in the arterial and venous system, respectively; then the device 12 is energized, so that in the suction stroke the piston 11, by opening the first check valve 2 and by closing the second check valve 6, extracts from the arterial system of the patient the blood to be treated, and delivers it through the suction tube 1 to

- 5 -

0149982

the manifold 3 wherefrom, as a consequence of the subsequent compression stroke of said piston with opening of the second check valve 6 and closing of the first check valve 2, the blood is pushed along the first portion 5 of the delivery tube 4, through the filter 7 and along the second portion 8 of the delivery tube 4 to be let in into the venous system in the scrubbed state.

From the above it can be easily appreciated that, contrary to similar known apparatus provided with peristaltic pumps, the blood during the extra-corporeal circulation as above said is not subjected to any traumatizing action, except a trauma, on the other hand neglectable, to which it is subjected when passing through the check valves 2 and 6.

It is possible, however, to eliminate also the above mentioned neglectable trauma in passing through the check valves by arranging in place of these latter two external pinchcocks,forming part of the fixed equipment of the machine, and which are actuated in synchronism with the motion of the piston 11 and, by acting on the section tube 1 and the delivery tube 4, respectively, do interrupt or allow alternately the passage of the blood in said tubes according to the section or compression stroke of said piston.

As a consequence of the particular arrangement of the device according to the present invention, it is possible, according to a modified embodiment, to provide a single access way to the above said circuit that operates alternately as inlet and outlet of the blood, this not being possible in conventional apparatus provided with peristaltic pumps unless two of these pumps are coupled together with the consequent inconveniences of structural complexity and increase of .costs.

In figure 2, where components equal to those already disclosed are identified with the same reference numerals and no additional description, there is shown an alternative embodiment as above where one can see that the free ends of the suction tube 1 and of the delivery tube 4 converge in a single tube 13 ending with a single needle-tube 14 to be inserted in the venous and arterial systems of the patient, systems that have been suitably shunted.

The above said arrangement constitutes a technical advantage of appreciable entity because it allows a single insertion of a needle-tube, in place of two, for each haemodialysis cycle.

With the embodiments of figures 1 and 2, the driving device 12 by acting on the exterior of the extra-corporeal circuit ensures the sterility and biological compatibility of the system.

As an alternative, the transmission of the reciprocating motion may occur at the interior of the extra-corporeal circuit, by replacing the syringe 10, 11 and the driving device 12, 12' by a suction-compression pump which acts upon an air cushion, previously sterilized, for instance by ultraviolet rays, ensuring again sterility and biological compatibility of the whole.

As hereinbefore recalled, and as it is desired to stress here, the qualifying aspect of the device according to the present invention resides in the absolute absence of traumatic actions, present in the known similar devices, that can cause the breakage of cellular components of the blood and for this reason also a prolonged and continued use of the same does not constitute a reason of damage for the blood itself.

One should remark also the "physiological" nature of the kind of pump according to the present invention that shows propelling characteristics similar to those of the human heart.

The adoption of a positive displacement pump according to the present invention allows also to monitor easily the flow rate of the blood in extra-corporeal circulation that is equal to the product of the displacement of the pump itself by the number of strokes of the piston in the unit of time.

Of course, as in the pumps presently used for the same purpose, there are provided conventional safety arrangements suitable for detecting and signalling possible operation malfunctions.

the present invention is not limited to the described embodiments, but encompasses any modified embodiment. So, for instance, in order to achieve the best hydraulic conditions, an external pinchcock may be arranged on
said portion 8 of the tube 4, near the connection
with the tube 13, whether using said valves or using
said external pinchcocks in place of valves.

## Claims

1. A pumping device for the extra-corporeal circulation of biological fluids, in particular blood, comprising a positive displacement pump system operatively connected with a circuit which is made with a sterile and biologically compatible material and comprises a flexible suction tube, a flexible delivery tube in fluid communication, between the two ends, with a therapeutic treatment apparatus, a manifold to which the first ends of said suction and delivery tubes are connected in fluid communication, connecting means to the body of a patient for the inlet into said suction tube and for the outlet from said delivery tube of said biological fluid, and means for the unidirectional flow of said biological fluid in said suction and delivery tubes.

2. A device according to claim 1, wherein said positive displacement pump system comprises a syringe-type instrument, of sterile and biologically compatible material, and a driving device releasably connected with the piston of said syringe-type instrument to impart a reciprocating motion to said piston.

3. A device according to claim 2, wherein said circuit and said syringe-type instrument form a disposable assembly.

4. A device according to claim 1, wherein said positive-displacement pump system consists of a suction-compression pump, acting upon an air cushion, constantly maintained in a sterile condition, through which the reciprocating motion of said pump is transmitted to said biological fluid.

5.  A device according to claim 1, wherein said connecting means to the body of the patient for the inlet into said suction tube and the outlet from said delivery tube of said biological fluid consist of two needle-tubes each of which positioned on the free end of a respective one of said suction and delivery tubes.

6.  A device according to claim 1, wherein said connecting means to the body of the patient for the inlet into said suction tube and for the outlet from said delivery tube of said biological fluid consist of a single needle-tube positioned at the free end of a tube the other end of which is fastened to the second ends of said suction and delivery tubes.

7.  A device according to claim 1, wherein said means for the unidirectional flow of said biological fluid in said suction and delivery tubes comprise a first and a second check valve, each arranged in a respective one of said suction and delivery tubes.

8.  A device according to claim 6, wherein said means for the unidirectional flow of said biological fluid in said suction and delivery tubes comprise three pinchcocks, mounted externally to said circuit, the one at said suction tube and the other two near the ends of the delivery tube, suitable for alternately interrupting the flow of said biological fluid in said tubes in synchronism with the motion of said positive-displace-ment pump system.

0149982

1/1

FIG.1

FIG.2